# FASCICULE DE BREVET EUROPEEN

(11) **EP 2 717 891 B1**
(45) Date de publication et mention de la délivrance du brevet: **14.09.2016**
(21) Numéro de dépôt: 12728639.1
(22) Date de dépôt: 14.05.2012
(51) Int. Cl.: A61K 36/185, A61K 31/366, A61K 31/7048, A61P 27/02

(54) **COMPOSITION DE BIXA ORELLANA POUR LE TRAITEMENT DE LA DÉGÉNÉRESCENCE MACULAIRE**
BIXA-ORELLANA-ZUSAMMENSETZUNG ZUR BEHANDLUNG VON MAKULADEGENERATION
BIXA ORELLANA COMPOSITION FOR TREATING MACULAR DEGENERATION& xA;

(30) Priorité: 13.05.2011 FR 1154172
(43) Date de publication de la demande: 16.04.2014
(73) Titulaire: Biophytis, 75001 Paris (FR); Université Pierre et Marie Curie (Paris 6), 75252 Paris Cedex 05 (FR)
(72) Inventeur: VEILLET, Stanislas, F-91600 Savigny Sur Orge (FR); LAFONT, René, F-75016 Paris (FR); FONTAINE, Valérie, F-75013 Paris (FR); SAHEL, José-Alain, F-75013 Paris (FR)
(74) Mandataire: Ipside
(86) Numéro de dépôt international: PCT/FR2012/000193
(87) Numéro de publication internationale: WO 2012/156600

(56) Documents cités:
- WO-A1-2010/149942
- WO-A2-01/85183
- TERASHIMA S ET AL: "Studies on aldose reductase inhibitors from natural products. IV. Constituents and aldose reductase inhibitory effect of Chrysanthemum morifolium, Bixa orellana and Ipomoea batatas.", CHEMICAL & PHARMACEUTICAL BULLETIN DEC 1991 LNKD- PUBMED:1814628, vol. 39, no. 12, décembre 1991 (1991-12), pages 3346-3347, XP002663845, ISSN: 0009-2363
- "Bixilia", website Biophytis , 8 novembre 2011 (2011-11-08), XP002663846, Extrait de l'Internet: URL:http://www.biophytis.com/en/ingredient s-sante/presentation-ingredients-sante/bix ilia.html [extrait le 2011-11-08]
- PRASAD P S ET AL: "Age-related macular degeneration: Current and novel therapies", MATURITAS, ELSEVIER SCIENCE PUBLISHERS IRELAND LTD, IR, vol. 66, no. 1, 1 mai 2010 (2010-05-01), pages 46-50, XP027009958, ISSN: 0378-5122 [extrait le 2010-04-15]
- Anonymous: "Medicinal Plants of the Guianas (Guyana, Surinam, French Guiana)", , 1 juillet 2008 (2008-07-01), pages 55-55, XP002680814, Extrait de l'Internet: URL:http://botany.si.edu/bdg/medicinal/Med icinal_plants_master.pdf [extrait le 2012-07-26]

## Description

L'invention concerne l'utilisation de composés et d'une composition pour prévenir certaines affections de la rétine.

L'invention a pour but d'améliorer la vision des individus atteints, ou au moins de stabiliser l'évolution de ces affections.

La dégénérescence maculaire liée à l'âge, ou DMLA, est une maladie dégénérative rétinienne chronique, évolutive et invalidante, qui touche le sujet âgé et dont l'origine est multifactorielle (Bellmann et Sahel, 2007). En France, c'est la première cause de malvoyance après 50 ans et on estime à un million le nombre de personnes concernées. Maladie à prédisposition génétique (Fajnkuchen et Cohen, 2008), elle est responsable d'un nombre croissant de cas de mal vision, proportionnel à l'augmentation de l'espérance de vie. Cette maladie atteint une petite partie de la rétine, la macula, zone qui sert à fixer les objets, à lire, à reconnaître les visages et à discerner les couleurs. La DMLA est très certainement polygénique avec l'intervention de facteurs de risque comme l'exposition prolongée à la lumière, l'hypertension artérielle, l'hypercholestérolémie et le tabagisme. Il existe deux formes de DMLA, la forme sèche ou atrophique qui représente 80% des cas, et la forme humide ou exsudative. Seule cette dernière, caractérisée par l'apparition de néovaisseaux derrière la rétine, peut désormais bénéficier de traitements.

Les mécanismes physiopathologiques de la DMLA sont encore peu connus, mais l'implication de processus d'intoxication aboutissant à la mort des cellules de l'épithélium pigmentaire rétinien (EPR) a été établie ces dernières années. En effet, au cours du vieillissement, ces cellules peuvent présenter des troubles de fonctionnement liés à l'accumulation dans des lysosomes de complexes protéo-lipidiques appelés granules de lipofuscine. Ces granules se forment progressivement par une accumulation de protéines et lipides non dégradés issus des segments externes des photorécepteurs phagocytés par l'EPR (Finnemann et al., 2002). La lipofuscine contient aussi des composés cytotoxiques dérivés du cycle visuel, tel l'A2E, qui est formé par addition de deux molécules de trans-rétinal avec une molécule d'éthanolamine. Sous l'effet de la lumière bleue, l'A2E s'oxyde et induit des oxydations protéiques, lipidiques et de l'ADN, provoquant ainsi un stress oxydatif important dans les cellules d'EPR en cours de vieillissement (Kim et al., 2006). Les tentatives de prévention ou de traitement de la forme sèche de la DMLA reposent sur une complémentation nutritionnelle avec des substances susceptibles de réduire l'accumulation et/ou les effets délétères de l'A2E (Dubernard et al., 2006 ; Souied et al., 2007 ; Dutot et al., 2008 ; Lecerf, 2009 ; Cohen et al., 2010 ; Lecerf et Desmettre, 2010).

Compte tenu du rôle très probable de ce mécanisme dans le développement de la DMLA, les inventeurs ont utilisé un modèle cellulaire de phototoxicité induite par l'association d'un traitement par l'A2E et d'une illumination par la lumière bleue sur des cultures primaires d'EPR dans lequel a été mesurée la survie cellulaire. Ce modèle, mis au point par l'Institut de la Vision, permet ainsi de réaliser le criblage de molécules visant à la découverte de nouveaux candidats pour un traitement de la forme sèche de la DMLA. Ce modèle original est plus proche de la situation « physiologique » que les lignées cellulaires couramment utilisées dans d'autres laboratoires (Dunn et al., 1996), car les cellules utilisées contiennent des substances protectrices apportées par l'alimentation de l'animal et ne sont donc pas en situation de « carence », et leur perturbation est provoquée par l'apport d'A2E.

L'invention prévoit ainsi de trouver un traitement alternatif à ceux déjà existants.

Plus précisément, les inventeurs ont découvert que l'incubation préalable des cellules avec certaines molécules permettait de diminuer fortement la mort cellulaire provoquée par une illumination avec des rayonnements bleus de cellules de l'EPR prétraitées avec l'A2E.

Selon l'invention, ces molécules sont présentes dans un extrait d'urucum, ou bien issues de dérivés de l'acide gallique ou de composés de la famille des anthocyanidines.

Un aspect de l'invention se rapporte donc à une composition comprenant un extrait de graines d'urucum, pour la photoprotection des cellules de l'épithélium pigmentaire rétinien chez le mammifère.

L'urucum, ou roucou, ou *Bixa orellana* est un arbre ou arbuste d'Amérique tropicale. Il donne des fruits rouges à épines remplis de graines.

Dans le cadre de l'invention, on entend par « extrait de graines de *Bixa orellana* » un extrait préparé à partir de la partie externe des graines, c'est-à-dire de la substance cireuse recouvrant les graines de *Bixa orellana.* Cette substance cireuse est connue pour être riche en bixine et en caroténoïdes, ainsi que pour son utilisation comme colorant alimentaire.

Il est connu du document WO 01/85183 une composition pour la prévention et le traitement des désordres oculaires, ladite compositon pouvant inclure un extrait de *Bixa orellana* en tant qu'inhibiteur de l'aldose réductase.

Cet extrait comprenant de l'acide gallique et/ou du pyrogallol, il s'agit vraisemblablement d'un extrait de feuilles de *Bixa orellana,* tel que décrit par Terashima et. al. (Chem. Pharm. Bull. 39(12), 3346-3347 (1991)), qui montre effectivement son activité en tant qu'inhibiteur de l'aldose réductase.

De plus, le document WO 01/85183 présente l'inhibition de l'aldose réductase comme faisant partie d'un mécanisme de protection contre la cataracte et la rétinopathie diabétique.

Le document WO 01/85183 ne montre donc pas l'efficacité d'un extrait de graines de *Bixa orellana* pour la photoprotection des cellules de l'EPR.

Selon un mode de réalisation de la présente invention, la composition à base de graines de *Bixa orellana* est destinée au traitement de la dégénérescence maculaire liée à l'âge (DMLA) chez le mammifère.

Selon un autre mode de réalisation de l'invention, la composition est destinée à traiter la maladie de Stargardt et/ou la rétinopathie pigmentaire. La maladie de Stargardt, ou syndrome de Stargardt, est une pathologie héréditaire, associant une baisse d'acuité visuelle bilatérale à une atrophie de la macula.

Selon un autre mode de réalisation de l'invention, la composition est destinée à prévenir les dommages à la rétine susceptibles d'être causés par l'exposition aux rayonnements bleus. Par rayonnements bleus, on entend les rayonnements correspondant à la bande bleue du spectre de la lumière visible, soit de longueur d'onde comprise entre 435 et 490 nm.

Selon un mode de réalisation de l'invention, la composition comprend en outre un dérivé de l'acide gallique et/ou un composé de la famille des anthocyanidines.

Le dérivé de l'acide gallique peut être l'acide ellagique, notamment purifié ou apporté sous forme d'un extrait de grenade. En effet, la grenade contient de l'acide ellagique en quantité importante (Panichayupakarananta et al., 2010).

Le composé de la famille des anthocyanidines peut être la cyanidine, notamment purifiée ou apportée sous forme d'un extrait d'açaï. Cette plante contient en effet des glycosides de cyanidine. La cyanidine peut également être apportée sous forme d'un extrait d'hibiscus.

La composition à base de graines de *Bixa orellana* peut être utilisée sous la forme d'un aliment, d'un complément alimentaire ou d'un médicament.

Par complément alimentaire, on entend un produit renfermant ledit composé ou ledit extrait ou enrichi en ledit composé ledit extrait ayant pour objet de compléter l'alimentation en apportant des nutriments bénéfiques pour la santé selon la définition donnée par la directive européenne 2002/46/EC. Par exemple, un complément alimentaire peut être une gélule ou comprimé à avaler ou une poudre ou petite ampoule à mélanger à un aliment et présentant des effets bénéfiques sur la rétine.

Par médicament, on entend un produit contenant une dose précise dudit composé ou dudit extrait selon la définition donnée par la directive européenne 65/65/CE à savoir toute substance ou composition présentée comme possédant des propriétés curatives ou préventives à l'égard des maladies humaines ou animales. Par exemple, le médicament contenant le composé aux doses thérapeutiques peut-être administré par voie orale sous forme de gélule ou comprimé ou injecté par voie intra-vitréenne ou tout autre voie permettant de conférer des effets bénéfiques sur la rétine.

Un autre aspect de l'invention se rapporte à une composition comprenant un dérivé de l'acide gallique et/ou un composé de la famille des anthocyanidines, pour la photoprotection des cellules de l'épithélium pigmentaire rétinien chez le mammifère.

Le dérivé de l'acide gallique est préférentiellement l'acide ellagique, notamment purifié ou apporté sous forme d'un extrait de grenade. Le composé de la famille des anthocyanidines est préférentiellement la cyanidine, notamment purifiée ou apportée sous forme d'un extrait d'açaï ou d'hibiscus.

Les applications de cette autre composition sont les mêmes que celles de la composition précédemment évoquée, comprenant un extrait de graines de *Bixa orellana.* De même que cette dernière, cette autre composition peut être utilisée sous la forme d'un aliment, d'un complément alimentaire ou d'un médicament.

L'invention sera mieux comprise à la lecture de la description qui suit et à l'examen des figures qui l'accompagnent. Celles-ci sont données à titre indicatif et nullement limitatif de l'invention.
La figure 1 illustre l'effet d'un extrait d'urucum, de la bixine et de la norbixine sur la protection de l'EPR soumis au test de phototoxicité.
La figure 2 illustre l'effet de l'acide chlorogénique, de la rutine et de l'acide ellagique sur la protection de l'EPR soumis au test de phototoxicité.
La figure 3 illustre l'effet de la cyanidine, de la cyanidine-3-glucoside, de la delphinidine-3-sambubioside et de la 20-hydroxyecdysone sur la protection de l'EPR soumis au test de phototoxicité.

### Exemples de réalisation de l'invention

### I. Préparation d'un extrait de Bixa orellana (extrait A)

L'extrait A est réalisé par agitation des graines d'urucum dans de l'éthanol absolu (3 L par Kg de graines) pendant 16 heures. L'agitation dans l'alcool a pour effet de détacher la pellicule cireuse se trouvant en surface des graines.

Une suspension est obtenue. Elle est passée au tamis afin d'éliminer les graines d'urucum. Cette suspension est ensuite réduite au 1/8^{ème}, puis décantée. Un dépôt solide se forme.

Le surnageant liquide, riche en lipides, est éliminé. Le dépôt solide est additivé de maltodextrine et séché par atomisation.

L'extrait A contient 16% en masse de bixine. Dans les exemples qui suivent, la concentration de l'extrait est exprimée en équivalent-bixine.

L'extrait A est également riche en caroténoïdes. Il contient également d'autres composés terpéniques, tels que le géranyl-géraniol et des tocotriénols (90% δ et 10% β), ainsi que plusieurs flavonoïdes.

L'extrait A présente les caractéristiques suivantes pour 100 g (tableau 1) :

**Tableau 1**

| | |
|---|---|
| Valeur énergétique | 319,04 Kcal |
| Glucides par différence | 38,10 g |
| Bixine | 16 g |
| Protéines | 7,7 g |
| Matière grasse par hydrolyse | 1,6 g |
| Fibres totales | 27,9 g |
| Sodium | 8,2 mg |
| Humidité | 6% |
| Matières minérales | 4,3 % |

Selon une variante de l'invention, l'extrait A peut être soumis à une saponification, de sorte à transformer tout ou partie de la bixine de l'extrait en norbixine.

### II. Tests d'activité

Les inventeurs ont testé 15 substances naturelles et l'extrait A sur un modèle cellulaire de phototoxicité de l'EPR décrit ci-après (tableau 2).

**Tableau 2**

| **Type** | **Nom du composé** | **Source (exemple)** |
|---|---|---|
| Témoins positifs | Lutéine | Epinard |
| | Zéaxanthine | Maïs |
| | Resvératrol | Raisin |
| Caroténoïdes | Bixine | Urucum |
| | Norbixine | " " |
| | extrait A | " " |
| | Crocétine | Safran |
| Acide phénolique | Acide chlorogénique | Maté |
| Flavone | Orientine | Açaï |
| Flavonol | Rutine | Sarrasin |
| Flavanone | Narinqénine | Citron |
| Benzypyrane | Acide eliagique | Grenade |
| Anthocyanines | Cyanidine 3-glucoside | Açaï |
| | Delphinidine 3-sambubioside | Hibiscus |
| Anthocyanidine | Cyanidine* | Açaï* |
| Stéroïde | 20-Hydroxyecdysone | Quinoa |

| | | |
|---|---|---|
| *La cyanidine est préparée après hydrolyse acide de ses formes glycosylées | | |

Afin de tester l'effet photoprotecteur des substances testées, les inventeurs ont utilisé un modèle cellulaire de phototoxicité induite par traitement par A2E suivi d'une illumination aux rayonnements bleus. Ce modèle a été réalisé à partir de cultures primaires d'EPR de porc adulte. La survie cellulaire a été déterminée par le rapport entre le nombre de cellules vivantes et le nombre total de cellules (vivantes + mortes, quantifiées respectivement à l'aide de colorations spécifiques). L'acquisition des images a été réalisée à l'aide d'un microscope à fluorescence piloté par le logiciel Metamorph et les quantifications ont été faites par traitement des images acquises par un programme de quantification dédié. Les expériences ont été réalisées dans des microplaques de 96 puits en quadruplicate et l'expérience a été reproduite au minimum quatre fois. Les cellules ont été traitées pendant 48 heures avec ces composés dont les dernières 24 heures en présence d'A2E, avant induction de la phototoxicité. Trois concentrations (0,1, 1 et 10 µM) ont été testées pour chaque composé. Certains composés ont fait l'objet de tests supplémentaires à 20 µM afin de réaliser une gamme de concentrations.

### III. Résultats

Les résultats, présentés sous forme de moyennes et écart-types, sont exprimés en pourcentage de survie par rapport au contrôle sans A2E.

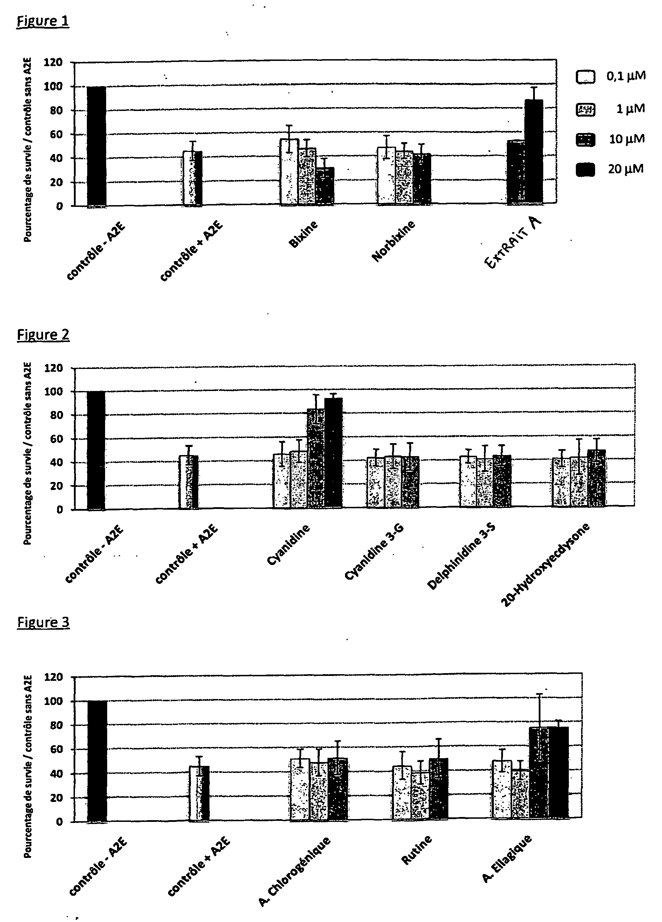

Les expériences n'ont pas montré d'effet protecteur des 15 composés ni de l'extrait aux concentrations de 0,1 et 1 µM (résultats non illustrés pour la lutéine, la zéaxanthine, le resvératrol, la crocétine, la naringénine et l'orientine et résultats illustrés pour l'extrait d'urucum, la cyanidine et l'acide ellagique figures 1 à 3).

Dans une série de tests (n=5), l'extrait A a permis d'observer une protection importante, avec une survie cellulaire à 20 µM de l'ordre de 93% de celle du contrôle sans A2E, à comparer avec celle du contrôle + A2E, qui n'est que de 45% (Figure 1).

Parmi les 15 composés testés à 10 µM, deux apportent une protection cellulaire contre la phototoxicité.

Notamment, à 10 µM, la cyanidine apporte une protection de l'ordre de 87% comparée au contrôle sans A2E. A 20 µM, ce même composé apporte une protection quasi-totale (Figure 2).

L'acide ellagique apporte également une protection de l'ordre de 68% comparée au contrôle sans A2E. A 20 µM, ce composé apporte une protection du même ordre que celle mesurée à 10 µM, mais avec une meilleure reproductibilité (Figure 3).

Les effets photoprotecteurs attendus pour la lutéine, la zéaxanthine et le resvératrol (« témoins positifs ») n'ont pas été observés dans ces conditions d'expérimentation.

### Références

Bellmann C, Sahel J-A. 2007. Aspects pathogéniques de la dégénérescence maculaire liée à l'âge (DMLA). J. Fr. Ophtalmol. 30 (hors-série 1), 1S11-1S16.
Cohen SY, Mauget-Faysse M, Oubraham H, Algan, M, Conrath J, Roquet W. 2010. Impact des habitudes nutritionnelles sur la pathologie maculaire évalué par mesure de la densité optique du pigment maculaire. J. Fr. Ophtalmol. 33, 234-240.
Dubemard G, Adam R, Proenca J, Offret H, Labetoulle M. 2006. Alimentation et dégénérescence maculaire l'iée à l'âge. NPG 6(33), 19-21.
Dunn KC, Aotaki-Keen AE, Putkey FR, Hjelmeland LM. 1996. ARPE-19, a human rétinal pigment épithélial cell line with differentiated properties. Exp. Eye Res. 62, 155-169.
Dutot M, Rambaux L, Warnet JM, Rat P. 2008. Modulation du stress oxydant par la myrtille riche en polyphénols sur un modèle de cellules humaines de rétine. J. Fr. Ophthalmol. 31(10), 975-980.
Fajnkuchen F, Cohen SY. Dégénérescence maculaire liée à l'âge et génétique : données actuelles. J. Fr. Ophtalmol. 31(6), 630-637.
Finnemann SC, Leung LW, Rodriguez-Boulan E. 2002. The lipofuscin component A2E selectively inhibits phagolysosomal dégradation of photoreceptor phospholipid by the retinal pigment epithelium. Proc. Nat. Acad. Sci. USA 99: 3842-2847.
Kim SR, Nakanishi K, Itagaki Y, Sparrow JR. 2006. Photooxidation of A2-PE, a photoreceptor outer segment fluorophore, and protection by lutein and zeaxanthin. Exp. Eye Res. 82, 828-839.
Lecerf J-M. 2009. Micronutriments : l'exemple de la dégénérescence maculaire liée à l'âge (DMLA). Médecine Maladies Métaboliques 3(5), 496-501.
Lecerf J-M, Desmettre T. 2010. Nutrition et dégénérescence maculaire liée à l'âge. J. Fr. Ophtalmol. 33, 749-757.
Panichayupakarananta P, Issuriya A, Sirikatitham A, Wang W. 2010. Antioxidant assay-guided purification and LC determination of ellagic acid in pomegranate peel. J. Chrom. Sci. 48, 456-459.
Souied E, Le Tien V, Coscas G, Soubrane G. 2007. Vers la prévention de la dégénérescence maculaire liée à l'âge. J. Fr. Ophtalmol. 30(5), 449-455.

## Revendications

1. Composition comprenant un extrait de graines de *Bixa orellana* pour la photoprotection des cellules de l'épithélium pigmentaire rétinien chez le mammifère.

2. Composition selon la revendication 1, pour son application dans le traitement de la dégénérescence maculaire liée à l'âge (DMLA) chez le mammifère.

3. Composition selon la revendication 1, pour son application dans le traitement de la maladie de Stargardt et/ou la rétinopathie pigmentaire.

4. Composition selon la revendication 1, pour prévenir les dommages à la rétine susceptibles d'être causés par l'exposition aux rayonnements bleus de longueur d'onde comprise entre 435 et 490 nm.

5. Composition selon l'une des revendications 1 à 4, comprenant un support acceptable pour être ingéré ou injecté dans l'oeil ou injecté dans le sang.

6. Composition selon l'une des revendications 1 à 5 en tant que médicament ou complément alimentaire ou aliment.

7. Composition selon l'une des revendications 1 à 6, comprenant en outre un dérivé de l'acide gallique et/ou un composé de la famille des anthocyanidines.

8. Composition selon la revendication 7, **caractérisée en ce que** le dérivé de l'acide gallique est l'acide ellagique.

9. Composition selon l'une des revendications 7 ou 8, **caractérisée en ce que** le composé de la famille des anthocyanidines est la cyanidine.

## Patentansprüche

1. Zusammensetzung, die einen Extrakt von *Bixaorellana*-Körnern umfasst, für den Lichtschutz des Epithelium pigmentosum der Retina beim Säuger.

2. Zusammensetzung nach Anspruch 1 zur Anwendung in der Behandlung der altersbedingten Makuladegeneration (AMD) beim Säuger.

3. Zusammensetzung nach Anspruch 1 zur Anwendung in der Behandlung des Stargardt-Syndroms und/oder von Pigment-Retinopathie.

4. Zusammensetzung nach Anspruch 1 zur Vorbeugung gegen Retinaschäden, die durch Exposition gegenüber blauer Strahlung mit einer Wellenlänge zwischen 435 und 490 nm verursacht werden können.

5. Zusammensetzung nach einem der Ansprüche 1 bis 4, die einen unbedenklichen Träger, um eingenommen oder in das Auge injiziert oder in das Blut injiziert zu werden, umfasst.

6. Zusammensetzung nach einem der Ansprüche 1 bis 5 als Arzneimittel oder Nahrungsmittelzusatz oder Nahrungsmittel.

7. Zusammensetzung nach einem der Ansprüche 1 bis 6, die weiterhin Gallussäurederivat und/oder eine Verbindung der Familie der Anthocyanidine umfasst.

8. Zusammensetzung nach Anspruch 7, **dadurch gekennzeichnet, dass** es sich bei dem Gallussäurederivat um Ellagsäure handelt.

9. Zusammensetzung nach einem der Ansprüche 7 oder 8, **dadurch gekennzeichnet, dass** es sich bei der Verbindung der Familie der Anthocyanidine um Cyanidin handelt.

## Claims

1. Composition comprising an extract of seeds of *Bixa orellana* for the photoprotection of the retinal pigment epithelium in a mammal.

2. Composition according to claim 1, for its application in the treatment of age-related macular degeneration (AMD) in a mammal.

3. Composition according to claim 1, for its application in the treatment of Stargardt's disease and / or retinitis pigmentosa.

4. Composition according to claim 1, for preventing retinal damage that could be caused by exposure to blue light of wavelength comprised between 435 and 490 nm.

5. Composition according to one of claims 1 to 4, comprising an acceptable carrier to be ingested or injected into the eye or injected into the blood.

6. Composition according to one of claims 1 to 5, as a medicament or food supplement or food.

7. Composition according to one of claims 1 to 6, further comprising a gallic acid derivative and / or a compound of the family of anthocyanidins.

8. Composition according to Claim 7, **characterized in that** the gallic acid derivative is ellagic acid.

9. The composition according to one of Claims 7 or 8, **characterized in that** the compound of the family of anthocyanidins is cyanidin.
